# EUROPEAN PATENT APPLICATION

(11) **EP 4 800 839 A1**
(43) Date of publication of application: **02.09.2026**
(21) Application number: 24898174.8
(22) Date of filing: 28.11.2024
(51) Int. Cl.: H01Q 1/24, H01Q 1/38, H01Q 9/04, G06F 1/16, H02J 50/00, A61B 5/024

(54) **WEARABLE ELECTRONIC DEVICE INCLUDING CONDUCTIVE PATTERN FOR ANTENNA**

(30) Priority: 01.12.2023 KR 20230172838; 17.01.2024 KR 20240007327
(71) Applicant: Samsung Electronics Co., Ltd., Suwon-si, Gyeonggi-do 16677 (KR)
(72) Inventor: LEE, Yoondo, Suwon-si Gyeonggi-do 16677 (KR); KIM, Taeik, Suwon-si Gyeonggi-do 16677 (KR); SHIN, Sanggon, Suwon-si Gyeonggi-do 16677 (KR); YEO, Hyungsok, Suwon-si Gyeonggi-do 16677 (KR); KIM, Gyusub, Suwon-si Gyeonggi-do 16677 (KR); OH, Youngjin, Suwon-si Gyeonggi-do 16677 (KR); LEE, Kyuho, Suwon-si Gyeonggi-do 16677 (KR); LEE, Yerim, Suwon-si Gyeonggi-do 16677 (KR)
(74) Representative: Gulde & Partner
(86) International application number: PCT/KR2024/019210
(87) International publication number: WO 2025/116580

(57) **Abstract**

A ring device according to an embodiment may comprise: a battery assembly including a conductive portion; a printed circuit board including a conductive pattern; and a wireless communication circuit electrically connected to the conductive pattern. For coupling, the conductive pattern may be spaced apart from and may face the conductive portion of the battery assembly. The wireless communication circuit may be configured to transmit or receive a radio frequency (RF) signal by using the conductive pattern and the battery assembly.

## Description

### [Technical Field]

The following descriptions relate to a wearable electronic device including a conductive pattern for an antenna.

### [Background Art]

A portable communication device may include various forms of devices such as a smart phone, a tablet, and a wearable device. Among them, the wearable device is gaining great popularity as it provides various interactions by being connected to another device such as the smart phone of a user, together with a function of tracking various biometric parameters such as a heart rate, a sleep pattern, and an activity level. As alternative wearable formfactors of wearable devices such as wristwatches, glasses, and clothing, a ring-type wearable device designed for the user to wear on a finger is being developed.

The above-described information may be provided as a related art for a purpose of helping understanding of the present disclosure. No claim or determination is raised as to whether any of the above-described descriptions may be applied as a prior art related to the present disclosure.

### [Disclosure]

### [Technical Solution]

A ring device according to an embodiment may include a battery assembly including a conductive portion, a printed circuit board including a conductive pattern, and a wireless communication circuit electrically connected to the conductive pattern. At least a portion of the conductive pattern may face and be spaced apart from the conductive portion of the battery assembly so as to enable coupling. The wireless communication circuit may be configured to transmit or receive a radio frequency (RF) signal using the conductive pattern and the battery assembly.

A wearable device according to an embodiment may include a housing forming a first curved surface that is in contact with a body of a user wearing the wearable device and a second curved surface opposite to the first curved surface, a battery disposed within the housing and including a case having a conductive portion, a printed circuit board disposed within the housing, including a flexible portion extending along the first curved surface and a conductive pattern formed on the flexible portion, and a wireless communication circuit electrically connected to the conductive pattern and disposed on the printed circuit board. The conductive pattern may include a first section and a second section extending from the first section and electrically connected to the wireless communication circuit. The first section of the conductive pattern may face and be spaced apart from the conductive portion of the battery for coupling. The wireless communication circuit may be configured to transmit or receive an RF signal using the conductive pattern and the battery.

### [Description of the Drawings]

FIG. 1 is a block diagram of an electronic device in a network environment according to various embodiments.
FIG. 2A is a diagram illustrating an exemplary ring device according to an embodiment.
FIG. 2B is an exploded view of an exemplary ring device according to an embodiment.
FIG. 3A is an exemplary diagram illustrating a printed circuit board according to an embodiment.
FIG. 3B is a diagram illustrating a region A of FIG. 3A.
FIG. 4A is an exemplary cross-sectional view of a ring device according to an embodiment.
FIG. 4B is a diagram illustrating a region B of FIG. 4A.
FIG. 4C is a diagram corresponding to a region C of FIG. 4A.
FIG. 5A is a diagram illustrating an antenna structure of a ring device according to an embodiment.
FIG. 5B is a diagram illustrating an antenna structure of a ring device according to an embodiment.
FIG. 6 is a diagram illustrating a ring device according to a comparative embodiment.
FIG. 7 is a diagram illustrating a ring device according to a comparative embodiment.
FIG. 8 is a graph illustrating radiation efficiency of a ring device according to an embodiment and ring devices according to a comparative embodiment.
FIG. 9 is a diagram illustrating an electric field distribution of a ring device according to an embodiment.
FIG. 10A illustrates a state in which a ring device according to an embodiment is worn on a human body phantom.
FIG. 10B illustrates a radiation pattern of a ring device according to an embodiment.
FIG. 11A is an exemplary cross-sectional view of a ring device according to an embodiment.
FIG. 11B is a diagram illustrating a region D of FIG. 11A.
FIG. 11C is a diagram illustrating a region D of FIG. 11A.
FIG. 12 is a diagram illustrating an antenna structure of a ring device according to an embodiment.
FIG. 13 is a graph illustrating radiation efficiency of ring devices according to an embodiment.
FIG. 14 is a diagram illustrating a ring device according to an embodiment.
FIG. 15A illustrates various examples of a conductive pattern according to an embodiment.
FIG. 15B illustrates various examples of a conductive pattern according to an embodiment.
FIG. 15C illustrates various examples of a conductive pattern according to an embodiment.
FIG. 16 is a graph illustrating radiation efficiency according to a conductive pattern according to an embodiment.

### [Mode for Invention]

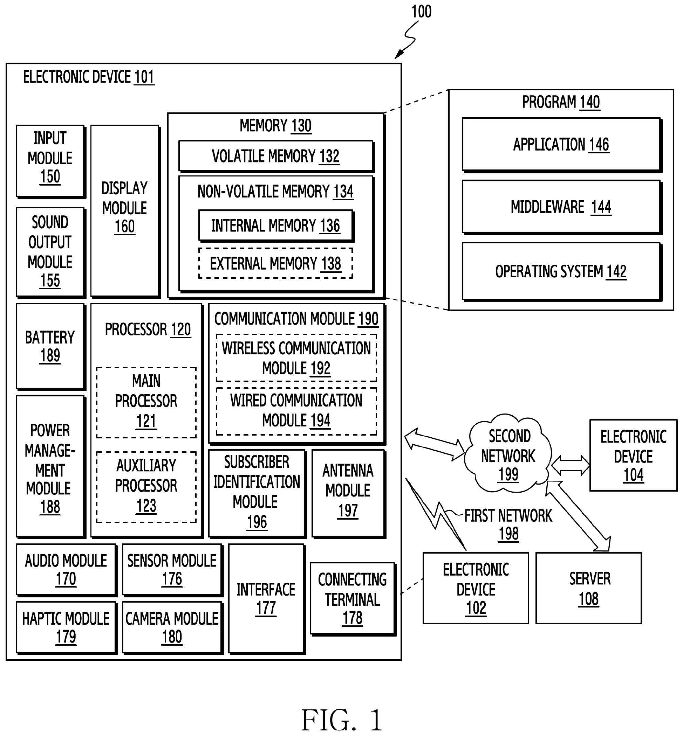

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

In the present disclosure, description of configurations having the same reference numeral referring to different drawings may be applied equally to each other unless otherwise noted, and overlapping descriptions thereof may be omitted.

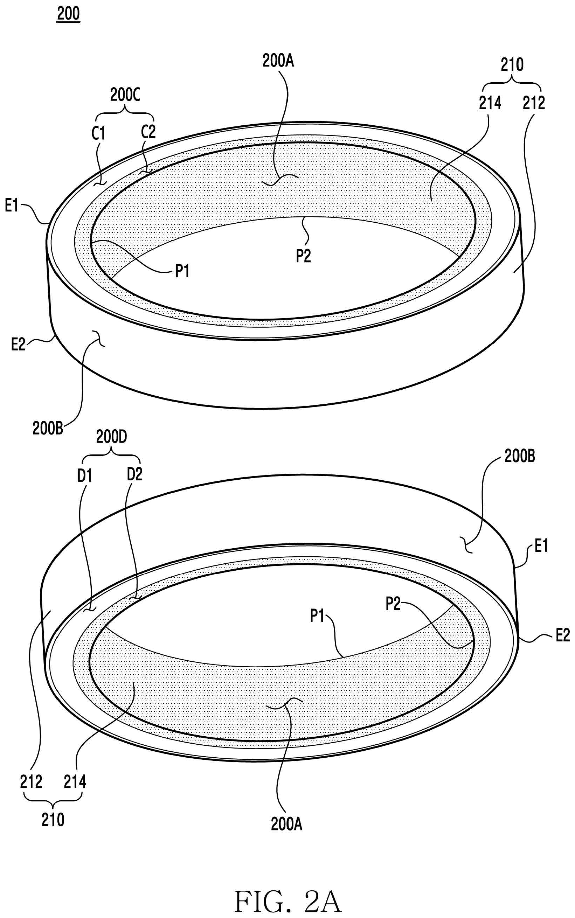

FIG. 2A is a diagram illustrating an exemplary ring device according to an embodiment. Referring to FIG. 2A, in an embodiment, a ring device 200 may include the electronic device 101 of FIG. 1. For example, the ring device 200 may include at least one of components of the electronic device 101.

The ring device 200 according to an embodiment may include a housing 210. The housing 210 may form an appearance of the ring device 200. For example, the housing 210 may define or include a first surface 200A, a second surface 200B, a third surface 200C, and a fourth surface 200D of the ring device 200.

In an embodiment, the first surface 200A may be a surface in contact with a body (e.g., a finger) of a user wearing the ring device 200. As a non-limiting example, the first surface 200A may be formed in a curved surface to correspond to a circumferential shape of the contacted finger. In an embodiment, the second surface 200B may be opposite to the first surface 200A. As a non-limiting example, the second surface 200B may include a curved surface.

In an embodiment, the third surface 200C may extend from a portion of the second surface 200B to a portion of the first surface 200A. For example, the third surface 200C may extend from a first edge E1 of the second surface 200B to a first edge P1 of the first surface 200A.

In an embodiment, the fourth surface 200D may be opposite to the third surface 200C. In an embodiment, it may extend from another portion (or a remaining portion) of the second surface 200B to another portion (or a remaining portion) of the first surface 200A. For example, the fourth surface 200D may extend from a second edge E2 of the second surface 200B to a second edge P2 of the first surface 200A.

In an embodiment, the housing 210 may include a first member 212 and a second member 214 coupled to the first member 212. In an embodiment, the first member 212 may form the second surface 200B of the housing 210, a first region C1 of the third surface 200C, and a first region D1 of the fourth surface 200D, but is not limited thereto. For example, the first region C1 of the third surface 200C and/or the first region D1 of the fourth surface 200D may be formed by the second member 214. In an embodiment, the first region C1 of the third surface 200C may extend from the first edge E1 of the second surface 200B, and the first region D1 of the fourth surface 200D may extend from the second edge E2 of the second surface 200B.

In an embodiment, the second member 214 may form the first surface 200A of the housing 210, a second region C2 of the third surface 200C, and a second region D2 of the fourth surface 200D, but is not limited thereto. For example, the second region C2 of the third surface 200C and/or the second region D2 of the fourth surface 200D may be formed by the first member 212. In an embodiment, the second region C2 of the third surface 200C may extend from the first edge P1 of the first surface 200A to the first region C1. The second region D2 of the fourth surface 200D may extend from the second edge P2 of the first surface 200A to the first region D1.

In an embodiment, the first member 212 may include a conductive material such as metal. For example, at least a portion of the first member 212 may be formed of the conductive material. As a non-limiting example, the portion of the first member 212 formed of metal may be used as an antenna radiator for transmitting and receiving a radio frequency (RF) signal. In an embodiment, the second member 214 may include a non-conductive portion such as plastic and/or a conductive portion formed of a conductive material such as metal.

In an embodiment, the ring device 200 may be referred to as an electronic device or a wearable device. In an embodiment, the second member 214 may be referred to as an inner ring in terms of forming the first surface 200A in contact with a body of the user, and the first member 212 may be referred to as an outer ring in terms of forming the second surface 200B opposite to the first surface 200A. In an embodiment, the first surface 200A may be referred to an inner surface of the ring device 200 (or the housing 210) in terms of contacting a body of the user wearing the ring device 200, and the second surface 200B may be referred to an outer surface of the ring device 200 (or the housing 210) in terms of opposing the first surface 200A. The third surface 200C and the fourth surface 200D surrounding a space between the first surface 200A and the second surface 200B may be referred to as a first side surface and a second side surface of the ring device 200 (or the housing 210), respectively. In an embodiment, the ring device 200 has been described as being worn on a finger, but is not limited thereto. For example, the ring device 200 may have a form of a bracelet or an anklet that may be worn on a wrist or an ankle.

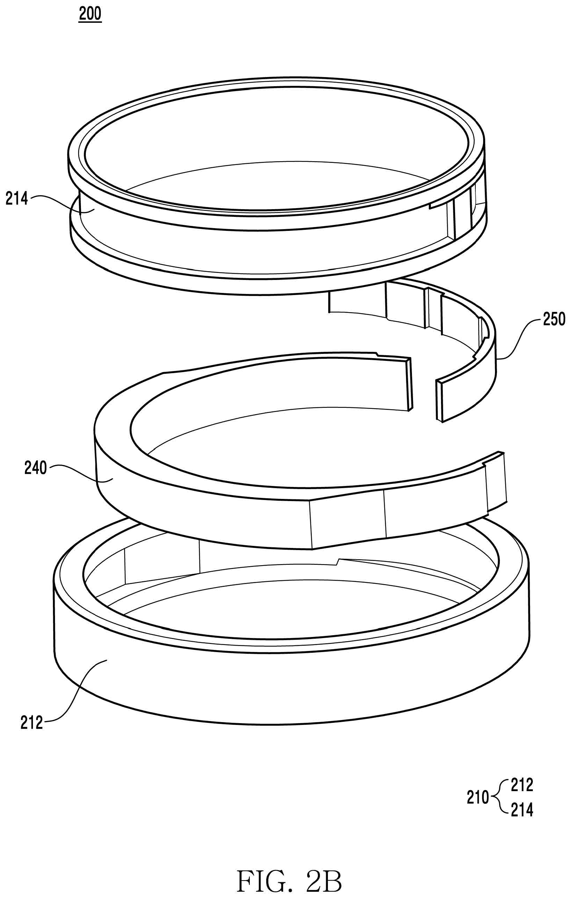

FIG. 2B is an exploded view of an exemplary ring device according to an embodiment. Referring to FIG. 2B, the ring device 200 according to an embodiment may include a printed circuit board 250 and a battery assembly 240.

In an embodiment, the printed circuit board 250 and the battery assembly 240 may be located inside the housing 210. For example, the printed circuit board 250 and the battery assembly 240 may be disposed between the first member 212 and the second member 214 of the housing 210. For example, the printed circuit board 250 and the battery assembly 240 may be supported by being surrounded by the first member 212 and/or the second member 214. In the present disclosure, 'Configuration A is supported by Configuration B' may include not only being supported by being in direct contact with the Configuration B, but also indirectly supported through another configuration between the Configuration A and the Configuration B.

In an embodiment, each of the printed circuit board 250 and the battery assembly 240 may be provided in a bent or bendable form at least a portion thereof to correspond to a shape of the ring device 200.

In an embodiment, various components of the ring device 200 may be disposed on the printed circuit board 250. As a non-limiting example, at least the processor 120, the memory 130, the sensor module 176, and the wireless communication module 192 of FIG. 1 may be disposed on the at least one printed circuit board.

According to an example, the battery assembly 240 and the printed circuit board 250 may be designed in a form of being bent according to a curved shape of the inside of the first member 212. The battery assembly 240 may be connected to the printed circuit board 250. For example, a corresponding connector of the battery assembly 240 may be coupled to a connector portion of the printed circuit board 250. The printed circuit board 250 and the battery assembly 240 coupled with each other may be fixed to the inside of the first member 212. Thereafter, the second member 214 may be formed through a molding process (e.g., epoxy molding). Thereafter, a surface treatment process such as polishing may be performed.

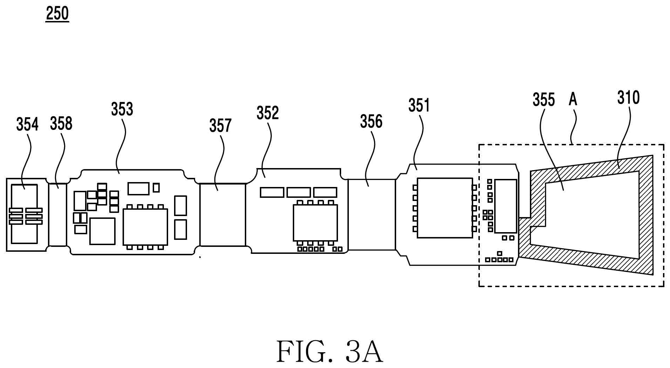

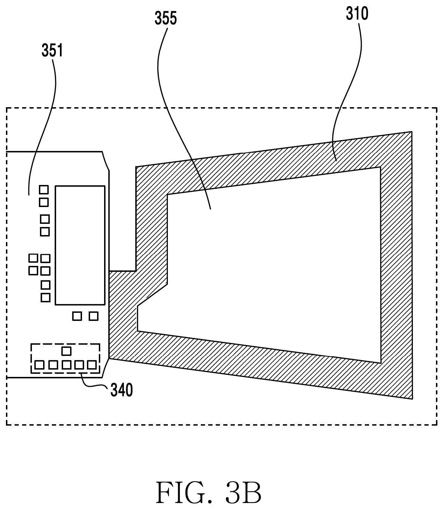

FIG. 3A is an exemplary diagram illustrating a printed circuit board according to an embodiment. FIG. 3B is a diagram illustrating a region A of FIG. 3A. Referring to FIGS. 3A and 3B, a printed circuit board 250 according to an embodiment may include a fifth portion 355. The fifth portion 355 may be located at an end of the printed circuit board 250. In an embodiment, the printed circuit board 250 may include a first portion 351 extending from the fifth portion 355. Additionally or optionally, the printed circuit board 250 may include at least one of portions 352, 353, 354, 356, 357, and 358 arranged in an order along a direction from the first portion 351.

In an embodiment, the first portion 351 may be spaced apart from the second portion 352. The sixth portion 356 may extend from the first portion 351 to the second portion 352. The first portion 351 and the second portion 352 may be connected through the sixth portion 356. The second portion 352 may be located between the first portion 351 and the third portion 353. In an embodiment, the third portion 353 may be spaced apart from the second portion 352. The seventh portion 357 may extend from the second portion 352 to the third portion 353. The second portion 352 and the third portion 353 may be connected through the seventh portion 357. The third portion 353 may be located between the second portion 352 and the fourth portion 354. In an embodiment, the fourth portion 354 may be spaced apart from the third portion 353. The eighth portion 358 may extend from the third portion 353 to the fourth portion 354. The third portion 353 and the fourth portion 354 may be connected through the eighth portion 358. In an embodiment, the fifth portion 355 may extend from the first portion 351. The first portion 351 may be located between the fifth portion 355 and the sixth portion 356.

In an embodiment, the printed circuit board 250 may be configured such that at least a portion thereof may be bent. For example, the fifth portion 355, the sixth portion 356, the seventh portion 357, and/or the eighth portion 358 of the printed circuit board 250 may be formed to be flexible.

In an embodiment, the first portion 351, the second portion 352, the third portion 353, and/or the fourth portion 354 of the printed circuit board 250 may be formed to be rigid. For example, on the first portion 351, the second portion 352, the third portion 353, and the fourth portion 354, various components of a ring device (e.g., the ring device 200 of FIG. 2A) may be disposed.

In an embodiment, a corresponding connector to which a connector of the battery assembly 240 of FIG. 2B is coupled may be disposed in another end of the printed circuit board 250 opposite to the fifth portion 355. For example, the corresponding connector may be disposed on the fourth portion 354.

In an embodiment, the fifth portion 355, the sixth portion 356, the seventh portion 357, and/or the eighth portion 358, which are formed to be flexible, may have a bent shape. The first portion 351 and the second portion 352 may be arranged at different angles through the flexible sixth portion 356. The second portion 352 and the third portion 353 may be arranged at different angles through the flexible seventh portion 357. The third portion 353 and the fourth portion 354 may be arranged at different angles through the flexible eighth portion 358. Accordingly, the printed circuit board 250 may have an entirely bent shape to correspond to a shape of a ring device 200.

In an embodiment, the printed circuit board 250 may include a conductive pattern 310 used as an antenna radiator, and a matching circuit 340 connected to the conductive pattern 310 to adjust impedance between the antenna radiator and a transmission line.

In an embodiment, the conductive pattern 310 may be formed or disposed on and/or in the fifth portion 355. The conductive pattern 310 may be formed of a material having electrical conductivity (e.g., metal such as copper). In an embodiment, the matching circuit 340 may be disposed on the first portion 351. The matching circuit 340 may include, for example, lumped elements such as a capacitor and an inductor, but is not limited thereto, and the matching circuit 340 may include various matching networks for adjusting impedance.

In an embodiment, a wireless communication circuit (e.g., the wireless communication module 192 of FIG. 1) of the ring device 200 may be electrically connected to the conductive pattern 310 through the matching circuit 340.

In an embodiment, the printed circuit board 250 may include a plurality of conductive layers extending from the first portion 351 to the eighth portion 358. As a non-limiting example, the first portion 351 of the printed circuit board 250 may include six conductive layers. As a non-limiting example, the first portion 351 may include first to sixth conductive layers located in an order from the lowermost layer. As a non-limiting example, the third conductive layer and the fourth conductive layer may extend beyond the first portion 351 to the fifth portion 355. As a non-limiting example, the conductive pattern 310 may be formed on the third conductive layer or the fourth conductive layer of the fifth portion 355.

In an embodiment, as described below, an area coupled with a conductive member (e.g., a battery 442, a first member 212, or a second member 214) facing the conductive pattern 310 may vary according to a shape (or an area) of the conductive pattern 310. Accordingly, characteristics (e.g., a resonance frequency) of an antenna using the conductive pattern 310 may vary. In FIGS. 3A and 3B, as an example for adjusting an amount of coupling and antenna characteristics accordingly, the conductive pattern 310 having a shape of a closed curve has been illustrated, but the shape of the conductive pattern 310 is not limited by the illustrated example.

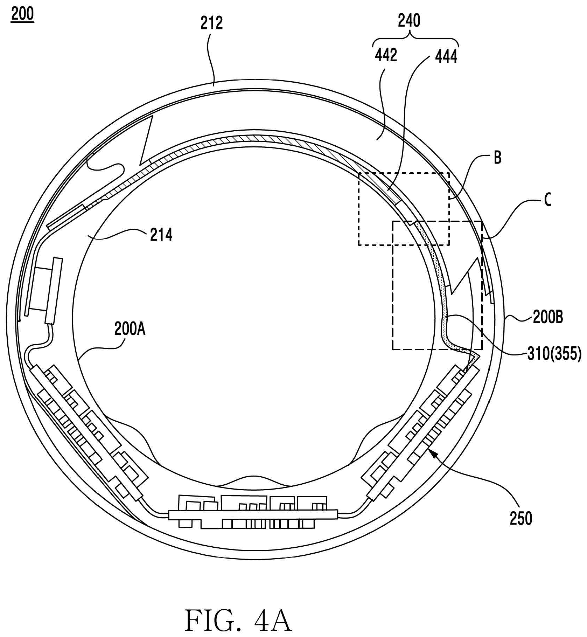

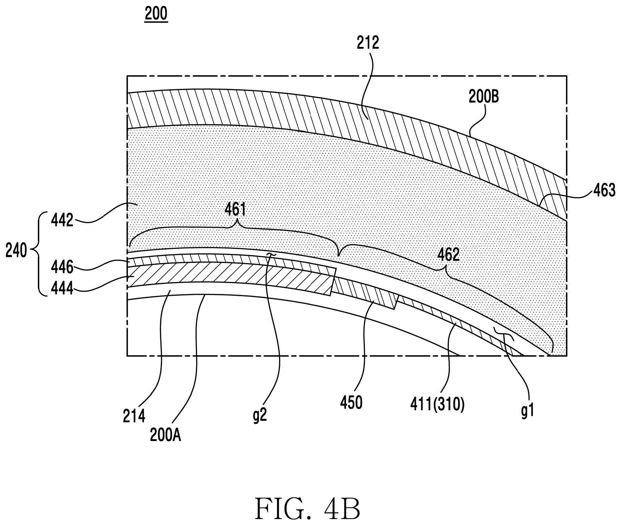

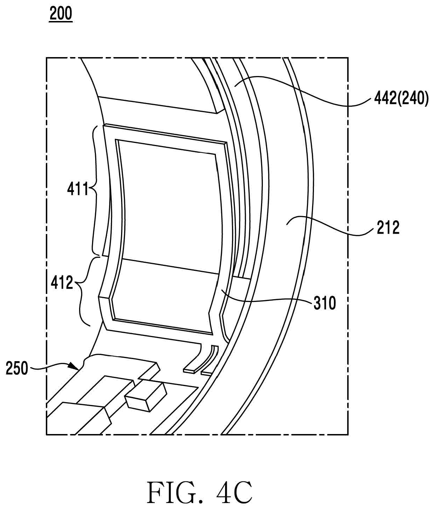

FIG. 4A is an exemplary cross-sectional view of a ring device according to an embodiment. FIG. 4B is a diagram illustrating a region B of FIG. 4A. FIG. 4C is a diagram corresponding to a region C of FIG. 4A.

Referring to FIGS. 4A and 4B, a battery assembly 240 according to an embodiment may include a battery 442 (e.g., the battery 189 of FIG. 1), an antenna 444, and a shielding member 446. The battery 442 may include, for example, a cell including a positive electrode, a negative electrode, an electrolyte, and a separator for a battery reaction, and a case in which the cell is accommodated. At least a portion of the case may be formed of a conductive material (e.g., metal). For example, at least a partial region of a surface of the case may be formed of the conductive material. In an embodiment, the battery 442 may include a conductive portion (e.g., the case or the surface of the case) coupled with the conductive pattern 310. For example, in the battery 442, the conductive portion (e.g., the case or the surface of the case) coupled with the conductive pattern 310 may be physically spaced apart.

In an embodiment, the antenna 444 may include, as a non-limiting example, an antenna for short-range communication. For example, the antenna 444 may include a coil (e.g., a wireless charging coil) for wirelessly transmitting and receiving power to and from an external device.

In an embodiment, the shielding member 446 may be located between the antenna 444 and the battery 442. The shielding member 446 may be spaced apart from the battery 442 through a gap g2. For example, an insulating member may be disposed in the gap g2. The insulating member may include an insulating tape for attaching the shielding member 446 and the battery 442. The shielding member 446 may include, as a non-limiting example, a graphite sheet.

A ring device 200 according to an embodiment may include an insulating tape 450 disposed between a fifth portion 355 of a printed circuit board 250 and the antenna 444. In order to prevent a short circuit between the antenna 444 and the conductive pattern 310 of the fifth portion 355, the insulating tape 450 may be located between an end of the antenna 444 and an end of the fifth portion 355. For example, the antenna 444 has been described as being included in the battery assembly 240, but alternatively or optionally, the antenna 444 may be included in the printed circuit board 250. For example, the antenna 444 may be disposed in the fifth portion 355 of the printed circuit board 250 or a portion extending from the fifth portion 355.

In an embodiment, the battery 442 (or the case of the battery 442) may include a first region 461, a second region 462, and a third region 463. The first region 461, the second region 462, and the third region 463 may form a portion of an appearance of the battery 442. The first region 461 and the second region 462 may face a first surface 200A (or a second member 214) of the ring device 200. The third region 463 may be opposite to the first region 461 and the second region 462 and may face a second surface 200B (or a first member 212) of the ring device 200. The first region 461 may overlap the antenna 444. For example, the first region 461 may support the antenna 444. The second region 462 may extend from the first region 461. The second region 462 may overlap the insulating tape 450 and the fifth portion 355. For example, the second region 462 may support the insulating tape 450 and the fifth portion 355.

Referring to FIG. 4C together with FIGS. 4A and 4B, in an embodiment, the fifth portion 355 of the printed circuit board 250 may at least partially overlap and be spaced apart from the battery assembly 240. The conductive pattern 310 of the fifth portion 355 may face and be spaced apart from the battery 442 of the battery assembly 240. For example, the conductive pattern 310 of the fifth portion 355 may face and be spaced apart from the conductive portion (e.g., the case) of the battery 442. For example, the conductive pattern 310 may at least partially overlap the battery 442. For example, with respect to a direction perpendicular to the first surface 200A, the conductive pattern 310 may at least partially overlap the battery 442.

In an embodiment, with respect to the direction perpendicular to the first surface 200A, the conductive pattern 310 may include a first section 411 overlapping the battery 442 and a second section 412 extending from the first section 411 in a direction away from the battery 442. The first section 411 may be located between the battery 442 and the first surface 200A. For example, the first section 411 may be supported by the second region 462 of the battery 442. As a non-limiting example, the second section 412 may include a step section. For example, the second section 412 may include a section closer to the second surface 200B than the first section 411. Through the step section, such as the first section 411 of the conductive pattern 310, a portion of the second section 412 may extend adjacent to the first surface 200A. In an embodiment, the second section 412 may be electrically connected to the wireless communication circuit.

In an embodiment, the first section 411 may be spaced apart from the battery 442 through a gap g1. For example, an insulating member may be disposed in the gap g1. The insulating member may include an insulating tape for attaching the fifth portion 355 of the printed circuit board 250 and the battery 442. The gap g1 may be, as a non-limiting example, approximately 0.1 mm or more and 0.3 mm or less. The gap g1 may be, as a non-limiting example, approximately 0.2 mm.

In an embodiment, the conductive pattern 310 and the conductive portion of the battery 442, facing each other and spaced apart from each other, may be coupled. The wireless communication circuit may transmit or receive an RF signal using the conductive pattern 310 and the battery 442 coupled to the conductive pattern 310.

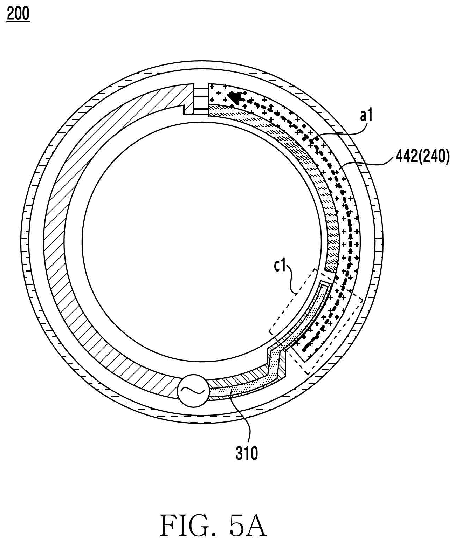

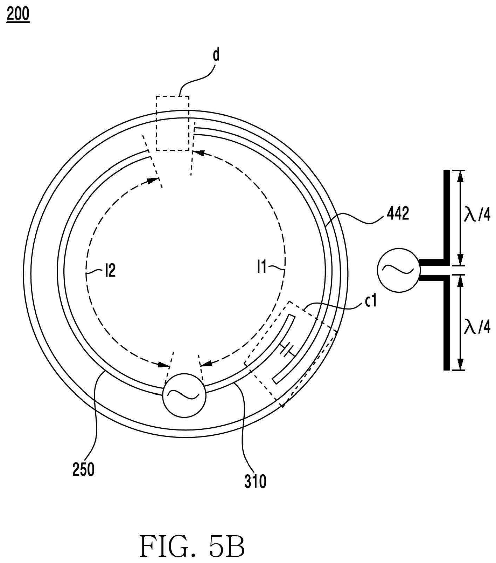

FIGS. 5A and 5B are diagrams illustrating an antenna structure of a ring device according to an embodiment.

Referring to FIG. 5A, a conductive pattern 310 and a battery 442 may be coupled in a region c1. The wireless communication circuit may feed into the conductive pattern 310. Electrical energy of the conductive pattern 310 may be transferred to the battery 442 through the coupled region c1. The conductive pattern 310 and the battery 442 coupled to the conductive pattern 310 may form an antenna radiator. Through the battery 442 coupled to the conductive pattern 310, a length of the antenna radiator may be increased as illustrated by an arrow a1. For example, a radiation area of the antenna radiator may be expanded. Accordingly, radiation performance of an antenna using the conductive pattern 310 and the battery 442 may be improved.

Referring to FIG. 5B, the conductive pattern 310 and the battery 442 may have a first length l1. The first length l1 may be, for example, approximately 1/4 of a wavelength λ of a communication frequency intended to be implemented through the antenna using the conductive pattern 310 and the battery 442. A remaining portion of a printed circuit board 250, except for the conductive pattern 310, may have a second length l2. As a non-limiting example, the second length l2 may be substantially the same as the first length l1. For example, the second length l2 may be approximately 1/4 of the wavelength λ of the communication frequency intended to be implemented. It may have equivalence to a dipole antenna in which a length of arms is implemented as λ/4, respectively. Accordingly, deterioration of antenna performance due to a lack of a ground area of the printed circuit board 250 may be reduced or prevented. For example, even when a mounting space for securing a ground area of a ring device 200 is insufficient, deterioration of antenna performance may be reduced or prevented.

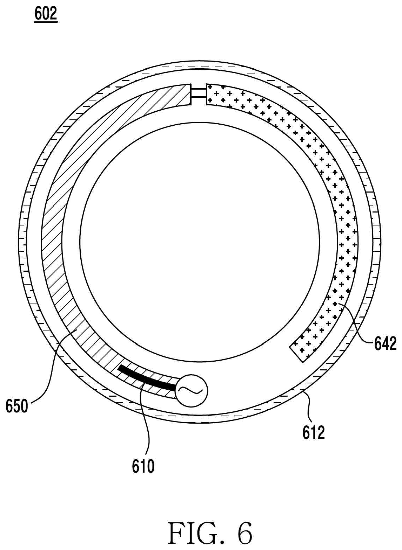

FIG. 6 is a diagram illustrating a ring device according to a comparative embodiment. Referring to FIG. 6, a ring device 602 according to a comparative embodiment includes a metal ring 612 (e.g., a first member 212) forming an appearance, a printed circuit board 650, an antenna pattern 610 formed on the printed circuit board 650, and a battery 642.

In order to reduce an influence on performance of the battery 642, the antenna pattern 610 of the ring device 602 of a comparative embodiment may be formed to be spaced apart from the battery 642. Due to a design constraint resulting therefrom and a small volume of the ring device 602 itself, the antenna pattern 610 of the ring device 602 according to a comparative embodiment may be difficult to satisfy a length for forming a resonance frequency. In addition, radiation performance of the antenna pattern 610 may be deteriorated by the metal ring 612.

On the other hand, a ring device (e.g., the ring device 200 of FIG. 4A) according to an embodiment may easily secure a length and performance of an antenna for forming a resonance frequency by using a conductive pattern 310 and a battery 442 coupled to the conductive pattern 310 as an antenna radiator.

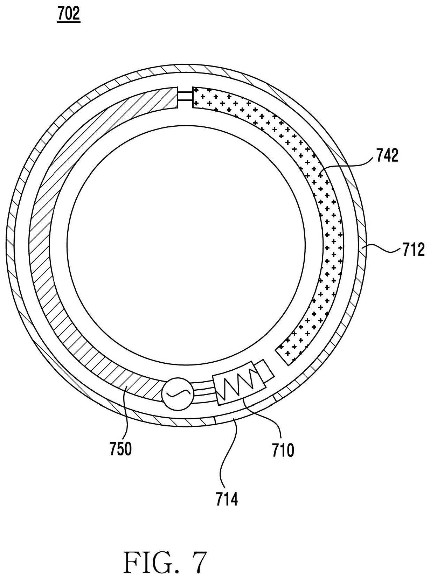

FIG. 7 is a diagram illustrating a ring device according to a comparative embodiment. Referring to FIG. 7, a ring device 702 according to a comparative embodiment includes a metal ring 712 (e.g., a first member 212) forming an appearance, a printed circuit board 750, a chip antenna 710 formed on the printed circuit board 750, and a battery 742.

In order to reduce an influence on performance of the chip antenna 710, the chip antenna 710 of the ring device 702 of a comparative embodiment may be formed avoiding the battery 742. Accordingly, according to a comparative embodiment, the chip antenna 710 of the ring device 702 may be difficult to satisfy a length for forming a resonance frequency. In addition, in order to prevent deterioration of radiation performance of the chip antenna 710 by the metal ring 712, the metal ring 712 may include a non-conductive portion 714 aligned with the chip antenna 710. The non-conductive portion 714 may cause an appearance design of the ring device 702 to be constrained, and an unnecessary process for manufacturing the non-conductive portion 714 may be accompanied.

On the other hand, the ring device (e.g., the ring device 200 of FIG. 4A) according to an embodiment may easily secure a length and performance of an antenna for forming a resonance frequency by using a conductive pattern 310 and a battery 442 coupled to the conductive pattern 310 as an antenna radiator. In addition, an unnecessary portion such as the non-conductive portion 714 may not be required in the first member 212 for radiation performance of the antenna radiator. Accordingly, a degree of design freedom may be improved, and an unnecessary process cost may be reduced.

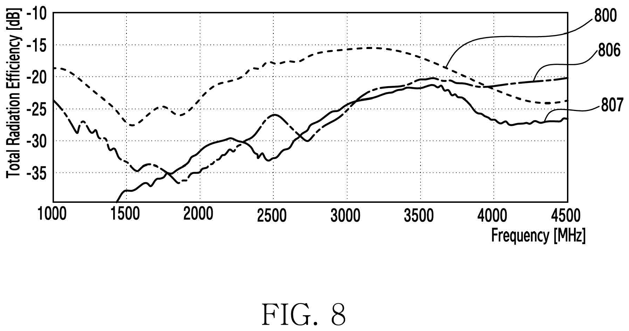

FIG. 8 is a graph illustrating radiation efficiency of a ring device according to an embodiment and ring devices according to a comparative embodiment. In FIG. 8, a graph 800 illustrates radiation efficiency of a ring device 200 according to an embodiment. A graph 806 illustrates radiation efficiency of a ring device 602 according to a comparative embodiment. A graph 807 illustrates radiation efficiency of a ring device 702 according to a comparative embodiment.

Referring to FIG. 8, the radiation efficiency of the graph 800 may be higher than the radiation efficiencies of the graphs 806 and 807 in a frequency band of 1,000 MHz to approximately 4,000 MHz. For example, the radiation efficiency of the graph 800 may be higher than the radiation efficiencies of the graphs 806 and 807 in a Bluetooth spectrum band of 2,400 MHz to 2,483.5 MHz.

Table 1 below shows a transmit power and a communication range of the ring device 200 according to an embodiment and the ring devices 602 and 702 according to a comparative embodiment.

**[Table 1]**

| Category | Ring device 200 according to an embodiment | Ring device 602 according to a comparative embodiment | Rind device 702 according to a comparative embodiment |
|---|---|---|---|
| Transmit power | -4 dBm | -1 dBm | -1 dBm |
| Communication range | 40 m | 34 m | 25 m |

Referring to Table 1, the ring device 200 according to an embodiment may have a decreased transmit power and an increased communication range than the ring devices 602 and 702 according to comparative embodiments. As the transmit power is decreased, current consumption of a battery may be reduced.

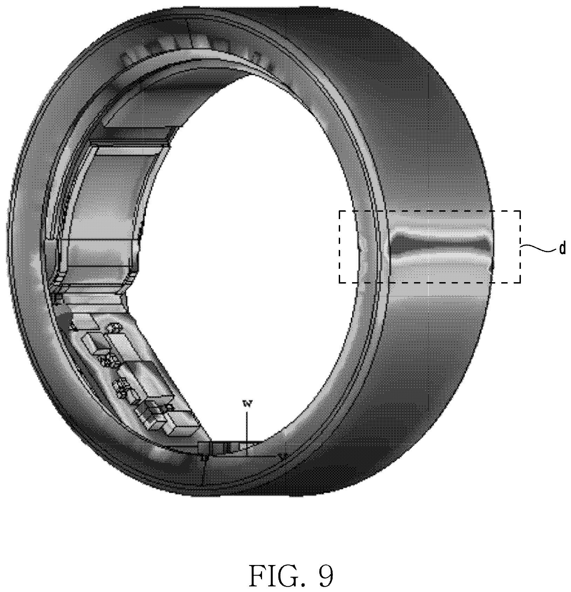

FIG. 9 is a diagram illustrating an electric field distribution of a ring device according to an embodiment. FIG. 9 may be an electric field distribution of a ring device 200 when an antenna radiator using the conductive pattern 310 and the battery 442 of FIG. 5B operates. Referring to FIG. 9 together with FIG. 5B, radiation energy may be concentrated in an end portion d of the battery 442. It may be because the conductive portion of the battery 442 coupled to the conductive pattern 310 operates as an antenna radiator.

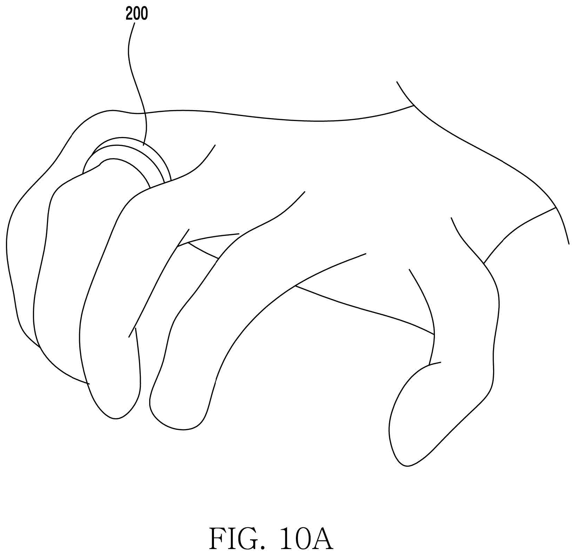

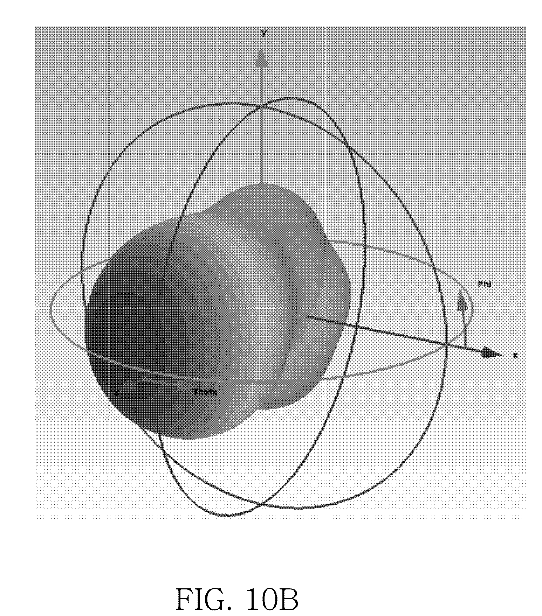

FIG. 10A illustrates a state in which a ring device according to an embodiment is worn on a human body phantom. FIG. 10B illustrates a radiation pattern of a ring device according to an embodiment. FIG. 10B illustrates a radiation pattern of the ring device in the state worn on the human body phantom of FIG. 10A. For example, a z direction of FIG. 10B may be a direction toward an end of a finger on which the ring device is worn. Referring to FIGS. 10A and 10B, the radiation pattern of the ring device according to an embodiment may have the largest radiation intensity toward a front direction of the finger.

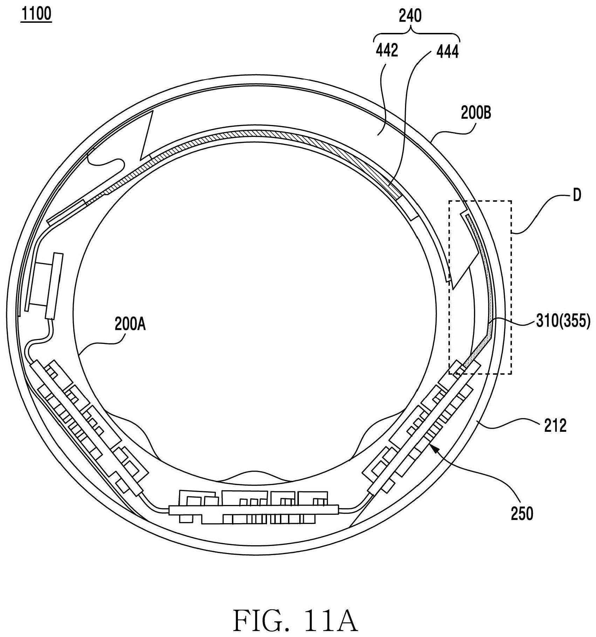

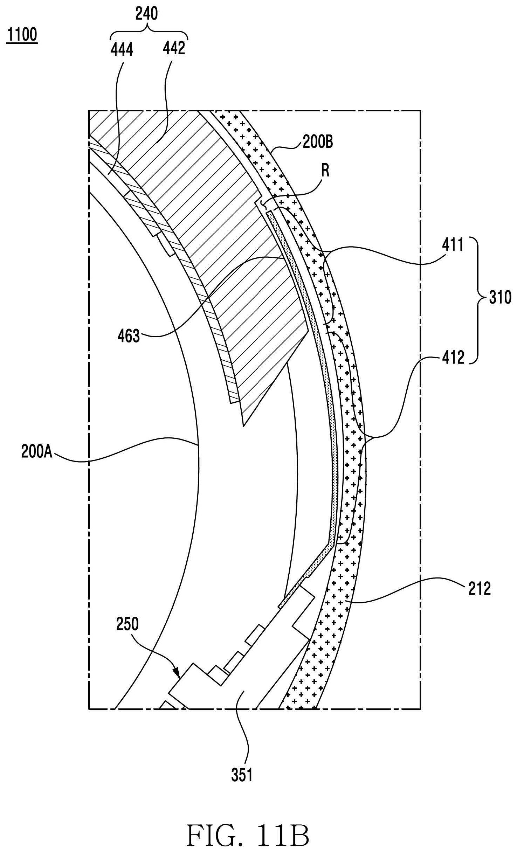

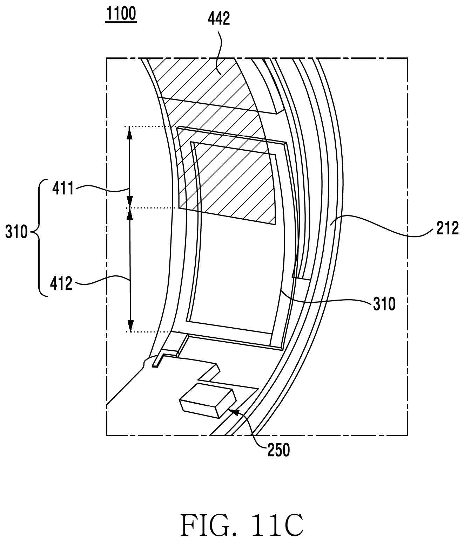

FIG. 11A is an exemplary cross-sectional view of a ring device according to an embodiment. FIGS. 11B and 11C are diagrams illustrating a region D of FIG. 11A.

Referring to FIGS. 11A, 11B, and 11C, a conductive pattern 310 of a ring device 1100 (e.g., the ring device 200 of FIG. 4A) may be disposed between a battery 442 and a first member 212, unlike the illustration of FIG. 4A. For example, a first section 411 of the conductive pattern 310 may be located between the battery 442 and a second surface 200B (or the first member 212) of the ring device 1100. The first section 411 may overlap the battery 442. For example, the first section 411 may overlap the battery 442 with respect to a direction perpendicular to the second surface 200B. The first section 411 may cover at least a portion of a third region 463 of the battery 442. For example, the first section 411 may be supported by the third region 463 of the battery 442. As a non-limiting example, the first section 411 may be located in a recess R formed in the third region 463 of the battery 442. An insulating member may be disposed between the first section 411 and the battery 442. The insulating member may include, for example, an insulating tape for attaching the first section 411 to the battery 442. In an embodiment, a fifth portion 355 (or the conductive pattern 310) of a printed circuit board 250 may extend along the second surface 200B of the first member 212. For example, an insulating material (e.g., resin) may be formed between the fifth portion 355 and the first member 212. As a non-limiting example, an insulating member (e.g., an insulating tape) may be disposed between the fifth portion 355 and the first member 212.

In an embodiment, the first section 411 may be supported by the first member 212. The first section 411 of the conductive pattern 310 may be covered by the first member 212. For example, the first section 411 of the conductive pattern 310 may overlap the first member 212. For example, the first section 411 may overlap the first member 212 with respect to a direction perpendicular to the second surface 200B.

In an embodiment, the conductive pattern 310 may be coupled with the battery 442 and the first member 212. In an embodiment, in order for the conductive pattern 310 and the conductive portion of the battery 442 to be coupled, the first section 411 may face and be spaced apart from the battery 442. In addition, in order for the conductive pattern 310 and the first member 212 to be coupled, the first section 411 may face and be spaced apart from the first member 212. In an embodiment, the first member 212 may be at least partially formed of a conductive material. For example, a region (or a portion) of the first member 212, facing the first section 411, may be formed of a conductive material. As a non-limiting example, the entire first member 212 may be formed of a conductive material. As a non-limiting example, a length of the first section 411 may be approximately 2 mm or more and 8 mm or less. As a non-limiting example, a length of the first section 411 may be approximately 5 mm. As a non-limiting example, a length of a second section 412 may be approximately 2 mm or more and 8 mm or less. As a non-limiting example, a length of the second section 412 may be approximately 5 mm. A first area in which the first section 411 faces the battery 442 may be substantially the same as a second area in which the first section 411 faces the first member 212, but is not limited thereto. For example, the first area and the second area may be different from each other. For example, the first area may be smaller than the second area. The first area and the second area may vary according to a communication frequency of an antenna.

In an embodiment, the wireless communication circuit may transmit or receive an RF signal using the conductive pattern 310, and the battery 442 and the first member 212 coupled to the conductive pattern 310.

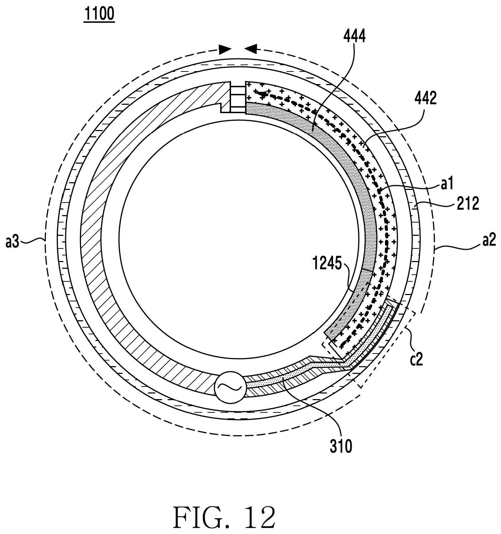

FIG. 12 is a diagram illustrating an antenna structure of a ring device according to an embodiment.

Referring to FIG. 12, a conductive pattern 310 may be coupled with a battery 442 and a first member 212 in a region c2. The wireless communication circuit may feed into the conductive pattern 310. Electrical energy of the conductive pattern 310 may be transferred to the battery 442 and the first member 212 through the coupled region c2. The conductive pattern 310, the battery 442, and the first member 212, which are coupled with each other, may form an antenna radiator. Through the coupling between the conductive pattern 310 and the battery 442, a length of the antenna radiator may be increased as illustrated by an arrow a1. In addition, through the coupling of the conductive pattern 310 and the first member 212, a length of the antenna radiator may be increased as illustrated by arrows a2 and a3. For example, a radiation area of the antenna radiator may be expanded. Accordingly, radiation performance of an antenna using the conductive pattern 310, the battery 442, and the first member 212 may be improved.

In addition, unlike the illustration of FIG. 5A, an antenna 444 may include an extension portion 1245. The extension portion 1245 may be located in a free space formed as a location of the conductive pattern 310 of FIG. 5A is changed to an opposite side of the battery 442. For example, the extension portion 1245 may overlap the battery 442 and the conductive pattern 310 (e.g., the first section 411 of FIG. 11C). The battery 442 may be partially located between the extension portion 1245 and the conductive pattern 310. As the antenna 444 further includes the extension portion 1245, transmission/reception performance (e.g., wireless charging performance) of the antenna 444 may be improved.

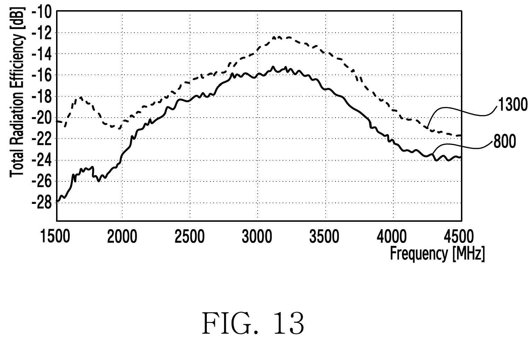

FIG. 13 is a graph illustrating radiation efficiency of ring devices according to an embodiment. In FIG. 13, a graph 800 illustrates radiation efficiency of a ring device 200 according to an embodiment. A graph 1300 illustrates radiation efficiency of a ring device 1100 according to an embodiment.

Referring to FIG. 13, the radiation efficiency of the graph 1300 may be higher than the radiation efficiency of the graph 800 in a frequency band of 1,500 MHz to 4,500 MHz. It may be because, unlike the ring device 200, a first member 212 is additionally used as an antenna radiator in a case of the ring device 1100.

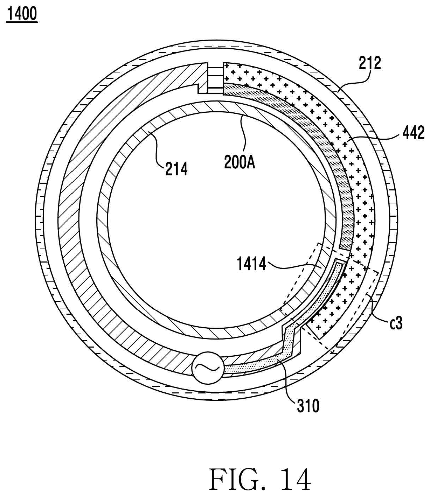

FIG. 14 is a diagram illustrating a ring device according to an embodiment. Referring to FIG. 14, a second member 214 of a ring device 1400 (e.g., the ring device 200 of FIG. 5A) according to an embodiment may include a conductive portion 1414. For example, the conductive portion 1414 may be formed of a conductive material (e.g., metal). The conductive portion 1414 may at least partially form a first surface 200A of the ring device 1400.

In an embodiment, a conductive pattern 310 may be disposed between a battery 442 and the conductive portion 1414. The conductive pattern 310 may be coupled to the battery 442 and the conductive portion 1414 in a region c3. The conductive pattern 310 may face and be spaced apart from the battery 442 for the coupling. The conductive pattern 310 may face and be spaced apart from the conductive portion 1414 of the second member 214 for the coupling.

In an embodiment, the wireless communication circuit may transmit or receive an RF signal using the conductive pattern 310, the battery 442 and the conductive portion 1414 coupled to the conductive pattern 310. As the battery 442 and the conductive portion 1414 are coupled to the conductive pattern 310, a radiation area of an antenna radiator may be expanded, and radiation performance may be improved.

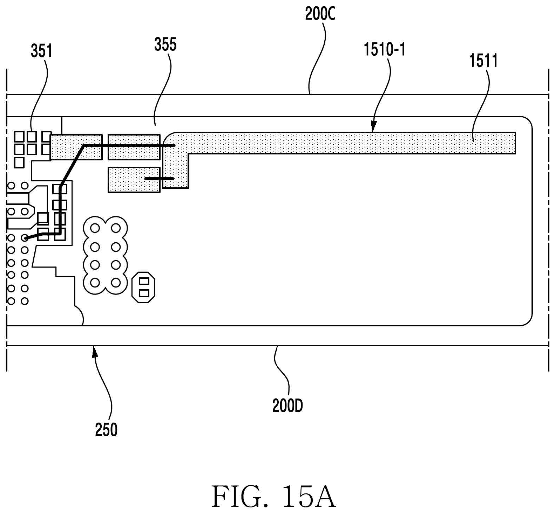

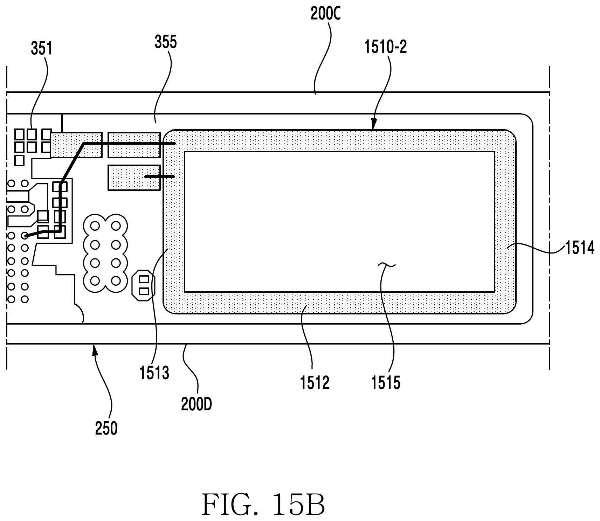

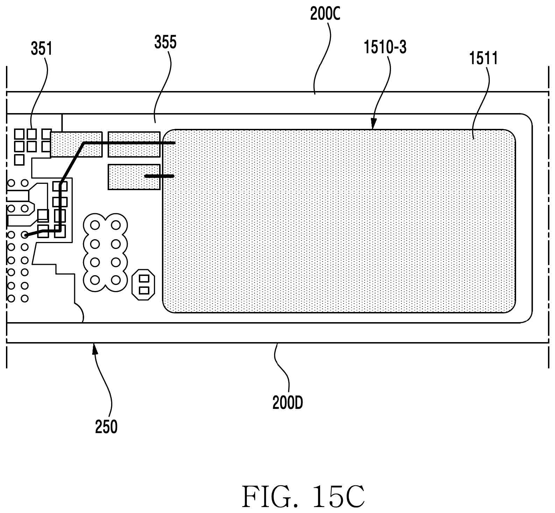

FIGS. 15A, 15B, and 15C illustrate various examples of a conductive pattern according to an embodiment. In FIGS. 15A, 15B, and 15C, various examples of a conductive pattern 310 are illustrated.

Referring to FIG. 15A, a conductive pattern 1501-1 may include a first pattern 1511. The first pattern 1511 may extend outside a first portion 351 of a printed circuit board 250 along a third surface 200C. As a non-limiting example, the first pattern 1511 may extend parallel to the third surface 200C. As a non-limiting example, the first pattern 1511 may extend substantially straight. The first pattern 1511 may be more adjacent to the third surface 200C than a fourth surface 200D. The conductive pattern 1501-1 may form an inverted F antenna (IFA).

Referring to FIG. 15B, a conductive pattern 1501-2 may have a substantially quadrangular shape. For example, the conductive pattern 1501-2 may have a quadrangular ring shape with an opening 1515 formed therein. For example, the conductive pattern 1501-2 may further include a second pattern 1512, a third pattern 1513, and a fourth pattern 1514 than the conductive pattern 1501-1.

The second pattern 1512 may extend outside the first portion 351 of the printed circuit board 250 along the fourth surface 200D. The second pattern 1512 may be more adjacent to the fourth surface 200D than the third surface 200C. The second pattern 1512 may be spaced apart from the first pattern 1511. As a non-limiting example, the second pattern 1512 may be substantially parallel to the first pattern 1511. For example, as illustrated in FIG. 3B, the conductive pattern 1510-2 may have a trapezoidal shape in which a distance between the first pattern 1511 and the second pattern 1512 increases as it is farther from the first portion 351.

The third pattern 1513 may extend from a first end of the first pattern 1511 to a first end of the second pattern 1512. The fourth pattern 1514 may extend from a second end of the first pattern 1511 to a second end of the second pattern 1512. As a non-limiting example, the fourth pattern 1514 may be substantially parallel to the third pattern 1513.

Referring to FIG. 15C, a conductive pattern 1510-3 may include a quadrangular patch. The conductive pattern 1510-3 may be a pattern in which the opening 1515 inside the conductive pattern 1510-2 of FIG. 15B is filled with a conductive material.

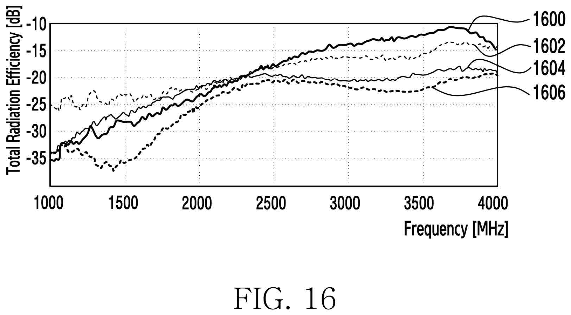

FIG. 16 is a graph illustrating radiation efficiency according to a conductive pattern according to an embodiment. In FIG. 16, radiation efficiency of the conductive patterns 1510-1, 1510-2, and 1510-3 of FIGS. 15A, 15B, and 15C is illustrated. For example, a graph 1600 may be radiation efficiency of the conductive pattern 1510-2 in a state of not being worn on a human body. A graph 1602 may be radiation efficiency of the conductive pattern 1510-2 in a state of being worn on the human body. A graph 1604 may be radiation efficiency of the conductive pattern 1510-3 in a state of being worn on the human body. A graph 1606 may be radiation efficiency of the conductive pattern 1510-1 in a state of being worn on the human body.

As an area of the conductive pattern increases, an influence due to the human body may increase, while a coupled area may also be increased. Referring to the graph 1600, in a frequency band of 2,400 MHz to 2483.5 MHz, radiation efficiency of the conductive pattern 1510-2 in a FREE state of not being worn on the human body may be the highest. Referring to the graph 1602, even when worn on the human body, the radiation efficiency of the conductive pattern 1510-2 may be substantially the same as the radiation efficiency (the graph 1600) in the FREE state. On the other hand, referring to the graphs 1604 and 1606, the radiation efficiency of the conductive pattern 1510-3 and the conductive pattern 1510-1 may be lower than the radiation efficiency (the graphs 1600 and 1602) of the conductive pattern 1510-2. It may be because a coupling amount is insufficient due to the too small area of the conductive pattern, or the influence of the human body is increased due to the too large area of the conductive pattern.

A ring device (e.g., the ring device 200 of FIG. 2A) according to an embodiment may include a battery assembly (e.g., the battery assembly 240 of FIG. 2B) including a conductive portion, a printed circuit board (e.g., the printed circuit board 250 of FIG. 3A) including a conductive pattern (e.g., the conductive pattern 310 of FIG. 3A), and a wireless communication circuit (e.g., the wireless communication module 192 of FIG. 1) electrically connected to the conductive pattern. The conductive pattern may face and be spaced apart from the conductive portion of the battery assembly for coupling. The wireless communication circuit may be configured to transmit or receive a radio frequency (RF) signal using the conductive pattern and the battery assembly.

In an embodiment, the ring device may include a first surface (e.g., the first surface 200A of FIG. 2A) that is in contact with a body of a user when the ring device is worn by the user and a second surface (e.g., the second surface 200A of FIG. 2A) that is opposite to the first surface. The conductive pattern may include a first section (e.g., the first section 411 of FIG. 4C) facing the conductive portion of the battery assembly. The first section may be located between the conductive portion and the first surface.

In an embodiment, the ring device may include an inner ring (e.g., the second member 214 of FIG. 14) including another conductive portion (e.g., the conductive portion 1414 of FIG. 14) and at least partially forming the first surface. The first section of the conductive pattern may face and be spaced apart from the another conductive portion of the inner ring for coupling. The wireless communication circuit may be configured to transmit or receive an RF signal using the conductive pattern, the battery assembly, and the inner ring.

In an embodiment, the conductive pattern may include a second section (e.g., the second section 412 of FIG. 4C) extending outside the battery assembly from the first section. The wireless communication circuit may be electrically connected to the second section.

In an embodiment, the second section may include a section closer to the second surface than the first section.

In an embodiment, the battery assembly may include a case at least partially including the conductive portion, and a wireless charging coil disposed on the case. The case may include a first region (e.g., the first region 461 of FIG. 4B) supporting the wireless charging coil and a second region (e.g., the second region 462 of FIG. 4B) extending from the first region and formed by the conductive portion. The first section of the conductive pattern may be supported by the second region of the case.

In an embodiment, the first region and the second region of the case may face the first surface.

In an embodiment, the ring device may include a first surface (e.g., the first surface 200A of FIG. 2A) that is in contact with a body of a user when the ring device is worn by the user and a second surface (e.g., the second surface 200B of FIG. 2A) that is opposite to the first surface. The conductive pattern may include a first section (e.g., the first section 411 of FIG. 11B) facing the conductive portion of the battery assembly. The first section may be located between the conductive portion and the second surface.

In an embodiment, the ring device may include an outer ring (e.g., the first member 212 of FIG. 11B) including another conductive portion and at least partially forming the second surface. The first section of the conductive pattern may face and be spaced apart from the another conductive portion of the outer ring for coupling. The wireless communication circuit may be configured to transmit or receive an RF signal using the conductive pattern, the battery assembly, and the outer ring.

In an embodiment, the conductive pattern may include a second section (e.g., the second section 412 of FIG. 11B) extending outside the battery assembly from the first section. The wireless communication circuit may be electrically connected to the second section.

In an embodiment, the second section may include a section closer to the first surface than the first section.

In an embodiment, the battery assembly may include a case at least partially including the conductive portion, and a wireless charging coil disposed on the case. The case may include a first region (e.g., the first region 461 of FIG. 4B) facing the first surface and a second region (e.g., the third region 463 of FIG. 11B) opposite to the first region and facing the second surface. The wireless charging coil may be supported by the first region of the case. The first section of the conductive pattern may be supported by the second region of the case.

In an embodiment, a portion of the wireless charging coil may overlap the first section of the conductive pattern.

In an embodiment, the ring device may include an insulating member disposed between the first section of the conductive pattern and the second region of the case.

In an embodiment, the printed circuit board may include a matching circuit (e.g., the matching circuit 340 of FIG. 3B), and the wireless communication circuit may be electrically connected to the conductive pattern through the matching circuit.

In an embodiment, an appearance of the ring device may include a first surface (e.g., the first surface 200A of FIG. 2A) that is in contact with a body of a user when the ring device is worn by the user, a second surface (e.g., the second surface 200B of FIG. 2A) opposite to the first surface, a third surface (e.g., the third surface 200C of FIG. 2A) extending from a first edge part of the first surface to a first edge part of the second surface, and a fourth surface (e.g., the fourth surface 200D of FIG. 2A) extending from a second edge part of the first surface to a second edge part of the second surface, and opposite to the third surface. The conductive pattern may include a first pattern (e.g., the first pattern 1511 of FIG. 15A) that extends substantially straight along the third surface and is closer to the third surface than the fourth surface.

In an embodiment, the conductive pattern may form an inverted F antenna (IFA).

In an embodiment, the conductive pattern may include a second pattern (e.g., the second pattern 1512 of FIG. 15B) extending along the fourth surface and facing the first pattern and being spaced apart from the first pattern, a third pattern (e.g., the third pattern 1513 of FIG. 15B) extending from a first end of the first pattern to a first end of the second pattern, and a fourth pattern (e.g., the fourth pattern 1514 of FIG. 15B) spaced apart from the third pattern and extending from a second end of the first pattern to a second end of the second pattern.

In an embodiment, the printed circuit board may include a rigid portion (e.g., the first portion 351 of FIG. 3A) on which the wireless communication circuit is disposed, and a flexible portion (e.g., the fifth portion 355 of FIG. 3A) on which the conductive pattern is formed.

In an embodiment, the wireless charging coil may be disposed on the printed circuit board.

A wearable device (e.g., the ring device 200 of FIG. 2A) according to an embodiment may include a housing (e.g., the housing 210 of FIG. 2A) forming a first curved surface (e.g., the first surface 200A of FIG. 2A) that is in contact with a body of a user wearing the wearable device and a second curved surface (e.g., the second surface 200B of FIG. 2A) opposite to the first curved surface, a battery (e.g., the battery 442 of FIG. 4A) disposed within the housing and including a case having a conductive portion, a printed circuit board (e.g., the printed circuit board 250 of FIG. 3A) disposed within the housing, including a flexible portion (e.g., the fifth portion 355 of FIG. 3A) extending along the first curved surface and a conductive pattern (e.g., the conductive pattern 310 of FIG. 3A) formed on the flexible portion, and a wireless communication circuit electrically connected to the conductive pattern and disposed on the printed circuit board. The conductive pattern may include a first section (e.g., the first section 411 of FIG. 4C) and a second section (e.g., the second section 412 of FIG. 4C) extending from the first section and electrically connected to the wireless communication circuit. The first section of the conductive pattern may face and be spaced apart from the conductive portion of the battery for coupling. The wireless communication circuit may be configured to transmit or receive a radio frequency (RF) signal using the conductive pattern and the battery.

In an embodiment, the housing may include a conductive member (e.g., the first member 212 of FIG. 11B) forming the second curved surface. The first section of the conductive pattern may be located between the conductive portion of the battery and the conductive member of the housing. The first section of the conductive pattern may face and be spaced apart from the conductive member of the housing for coupling. The wireless communication circuit may be configured to transmit or receive a radio frequency (RF) signal using the conductive pattern, the battery, and the conductive member.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A ring device (200) comprising:
a battery assembly (240) including a conductive portion;
a printed circuit board (250) including a conductive pattern (310); and
a wireless communication circuit (192) electrically connected to the conductive pattern (310),
wherein the conductive pattern (310) faces and is spaced apart from the conductive portion of the battery assembly (240) for coupling, and
wherein the wireless communication circuit (192) is configured to transmit or receive a radio frequency (RF) signal using the conductive pattern (310) and the battery assembly (240).

2. The ring device (200) of claim 1, comprising:
a first surface (200A) that is in contact with a body of a user when the ring device (200) is worn by the user and a second surface (200B) that is opposite to the first surface (200A),
wherein the conductive pattern (310) includes a first section (411) facing the conductive portion of the battery assembly (240), and
wherein the first section (411) is located between the conductive portion and the first surface (200A).

3. The ring device (200) of claim 2, comprising:
an inner ring (214) comprising another conductive portion (1414) and at least partially forming the first surface (200A),
wherein the first section (411) of the conductive pattern (310) faces and is spaced apart from the another conductive portion (1414) of the inner ring (214) for coupling, and
wherein the wireless communication circuit (192) is configured to transmit or receive an RF signal using the conductive pattern (310), the battery assembly (240), and the inner ring (214).

4. The ring device (200) of claim 2 or claim 3, wherein:
the conductive pattern (310) includes a second section (412) extending outside the battery assembly (240) from the first section (411); and
the wireless communication circuit (192) is electrically connected to the second section (412).

5. The ring device (200) of claim 4,
wherein the second section (412) includes a section closer to the second surface (200B) than the first section (411).

6. The ring device (200) of any one of claims 2 to 5,
wherein the battery assembly (240) includes:
a case at least partially including the conductive portion; and
a wireless charging coil (444) disposed on the case,
wherein the case includes a first region (461) supporting the wireless charging coil (444) and a second region (462) extending from the first region (461) and formed by the conductive portion, and
wherein the first section (411) of the conductive pattern (310) is supported by the second region (462) of the case.

7. The ring device (200) of claim 6,
wherein the first region (461) and the second region (462) of the case face the first surface (200A).

8. The ring device (200) of claim 1, comprising:
a first surface (200A) that is in contact with a body of a user when the ring device (200) is worn by the user and a second surface (200B) that is opposite to the first surface (200A),
wherein the conductive pattern (310) includes a first section (411) facing the conductive portion of the battery assembly (240), and
wherein the first section (411) is located between the conductive portion and the second surface (200B).

9. The ring device (200) of claim 8, comprising:
an outer ring (212) including another conductive portion (1414) and at least partially forming the second surface (200B),
wherein the first section (411) of the conductive pattern (310) faces and is spaced apart from the another conductive portion (1414) of the outer ring (212) for coupling, and
wherein the wireless communication circuit (192) is configured to transmit or receive an RF signal using the conductive pattern (310), the battery assembly (240), and the outer ring (212).

10. The ring device (200) of claim 8 or claim 9,
wherein the conductive pattern (310) includes a second section (412) extending outside the battery assembly (240) from the first section (411), and
wherein the wireless communication circuit (192) is electrically connected to the second section (412).

11. The ring device (200) of claim 10,
wherein the second section (412) includes a section closer to the first surface (200A) than the first section (411).

12. The ring device (200) of any one of claims 8 to 11,
wherein the battery assembly (240) includes:
a case at least partially including the conductive portion; and
a wireless charging coil (444) disposed on the case,
wherein the case includes a first region (461) facing the first surface (200A) and a second region (462) opposite to the first region (461) and facing the second surface (200B),
wherein the wireless charging coil (444) is supported by the first region (461) of the case,
wherein the first section (411) of the conductive pattern (310) is supported by the second region (462) of the case.

13. The ring device (200) of claim 12,
wherein a portion of the wireless charging coil (444) overlaps the first section (411) of the conductive pattern (310).

14. The ring device (200) of any one of claims 6, 7, and 12, comprising:
an insulating member disposed between the first section (411) of the conductive pattern (310) and the second region (462) of the case.

15. The ring device (200) of any one of claims 1 to 14, wherein:
the printed circuit board (250) includes a matching circuit (340); and
the wireless communication circuit (192) is electrically connected to the conductive pattern (310) through the matching circuit (340).
